# EUROPÄISCHE PATENTSCHRIFT

(11) **EP 1 527 059 B1**
(45) Veröffentlichungstag und Bekanntmachung des Hinweises auf die Patenterteilung: **22.02.2006**
(21) Anmeldenummer: 03766328.3
(22) Anmeldetag: 28.07.2003
(51) Int. Cl.: C07D 301/12, C07D 303/02

(54) **VERFAHREN ZUR HERSTELLUNG VON PROPENYLOXID UNTER VERWENDUNG EINES NACHREAKTORS MIT MEHREREN EINSPEISE- UND/ODER ABLAUFSTELLEN**
METHOD FOR PRODUCING PROPENYL OXIDE USING A SECONDARY REACTOR COMPRISING SEVERAL FEED AND/OR OUTLET POINTS
PROCEDE DE PRODUCTION D'OXYDE DE PROPYLENE A L'AIDE D'UN REACTEUR COMPLEMENTAIRE COMPORTANT PLUSIEURS POINTS D'ALIMENTATION ET/OU D'EXPLOITATION

(30) Priorität: 29.07.2002 DE 10234448
(43) Veröffentlichungstag der Anmeldung: 04.05.2005
(73) Patentinhaber: BASF Aktiengesellschaft, 67056 Ludwigshafen (DE)
(72) Erfinder: GÖBBEL, Hans-Georg, 67169 Kallstadt (DE); BASSLER, Peter, 68519 Viernheim (DE); TELES, Joaquim, Henrique, 67166 Otterstadt (DE); RUDOLF, Peter, 68526 Ladenburg (DE)
(74) Vertreter: Isenbruck, Günter
(86) Internationale Anmeldenummer: PCT/EP2003/008317
(87) Internationale Veröffentlichungsnummer: WO 2004/013116

(56) Entgegenhaltungen:
- EP-A- 1 266 892
- WO-A-00/07965
- US-A- 5 349 072

## Beschreibung

Die vorliegende Erfindung betrifft ein Verfahren zur katalytischen Umsetzung einer organischen Verbindung mit einem Hydroperoxid zu einem Oxiran, wobei im Laufe des Verfahrens Hydroperoxid abgetrennt und erneut mit der organischen Verbindung in einem Rohrreaktor umgesetzt wird, der mindestens zwei Einspeisestellen und/oder zwei Ablaufstellen besitzt. Durch das neue Verfahren kann die Standzeit des als Festbettreaktor ausgeführten Rohrreaktors bezüglich seiner katalytischen Aktivität vorteilhaft erhöht werden. Insbesondere kann nach dem erfindungsgemäßen Verfahren Propylenoxid aus Propylen und Wasserstoffperoxid in Anwesenheit von Methanol als Lösungsmittel hergestellt werden, wobei die Umsetzung an einem Titan-haltigen Zeolith erfolgt.

Nach den gängigen Verfahren des Standes der Technik werden Oxirane durch Umsetzung geeigneter organischer Verbindungen mit Hydroperoxiden hergestellt, wobei diese Umsetzungen einstufig oder mehrstufig durchgeführt werden können.

Beispielsweise sieht das in der WO 00/07965 beschriebene mehrstufige Verfahren vor, dass die Umsetzung der organischen Verbindung mit einem Hydroperoxid wenigstens die Schritte (i) bis (iii) umfasst:
(i) Umsetzung des Hydroperoxids mit der organischen Verbindung unter Erhalt eines Produktgemisches, umfassend die umgesetzte organische Verbindung und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit der organischen Verbindung.

Demgemäss findet die Umsetzung der organischen Verbindung mit dem Hydroperoxid in mindestens zwei Stufen (i) und (iii) statt, wobei das in Stufe (ii) abgetrennte Hydroperoxid erneut in die Reaktion eingesetzt wird.

Vorzugsweise erfolgen dabei die Umsetzungen in den Stufen (i) und (iii) in zwei getrennten Reaktoren, etwa Festbettreaktoren, wobei vorzugsweise die Umsetzung der Stufe (i) in einem isothermen und die Umsetzung der Stufe (iii) in einem adiabatischen Reaktor stattindet. Der in Stufe (i) verwendete Reaktor wird nachfolgend auch als Hauptreaktor und der in Stufe (iii) verwendete Reaktor als Nachreaktor bezeichnet. Dem offenbarten Verfahren zufolge wird dabei dem Nachreaktor das das Hydroperoxid und die organische Verbindung umfassende Reaktionsgemisch über lediglich eine Einspeisestelle, die beispielsweise am Boden des Nachreaktors angebracht sein kann, zugeführt. Das Produktgemisch wird lediglich über eine Ablaufstelle, die beispielsweise am Kopf des Reaktors angebracht ist, aus dem Reaktor entnommen.

Allgemein kann dieses mehrstufige Verfahren zur Umsetzung von Alkenen als organischer Verbindung mit Hydroperoxiden zu Oxiranen verwendet werden. Vorzugsweise wird bei dieser Sequenz die besagte Umsetzung auch an einem heterogenen Katalysator durchgeführt.

Als heterogene Katalysatoren können beispielsweise Zeolithe verwendet werden, vorzugsweise titanhaltige Zeolithe, wie z. B. der Silicalit TS-1.

Es ist jedoch bekannt, dass die katalytische Aktivität dieser Katalysatoren während der Reaktion infolge Belegung mit Edukten oder Produkten mit der Betriebszeit abnehmen kann.

Diese Abnahme der Aktivität kann insbesondere dann problematisch werden, wenn die Reaktion in einem Festbettreaktor durchgeführt wird. Man kann hier nämlich den Katalysator, der an das Festbett gebunden ist, nicht laufend austauschen oder reaktivieren, da dies stets mit einer den Herstellprozess störenden Unterbrechung verbunden ist.

Um dieser Abnahme der katalytischen Aktivität der Katalysatoren, insbesondere titanhaltiger Zeolithe, wie etwa des Zeoliths TS-1, entgegen zu wirken, wurde deshalb bereits vorgeschlagen, die Reaktionstemperatur oder den pH-Wert des Reaktionsgemisches oder beide gleichzeitig den sich verändernden Reaktionsbedingungen anzupassen (WO 01/10855).

Bekannt sind auch Methoden zur Reaktivierung der vorstehend genannten Katalysatoren, wie sie beispielsweise in der DE-A 197 23 949.8 beschrieben sind. Während der Regenerierung muss dann jedoch die Umsetzung der organischen Verbindung mit dem Hydroperoxid unter Oxiran-Bildung im Reaktor unterbrochen werden. Da die Regenerierung des Katalysators im Hauptreaktor relativ häufig notwendig ist, ist auch bereits bekannt, diesen Reaktor mindestens zweifach auszuführen (WO 01/72729). Unter taktweiser Schaltung der Reaktoren kann dann dennoch ein günstiges wirtschaftliches Verfahren, wie es etwa ein kontinuierlicher Prozess bietet, zur Anwendung gelangen.

Bei der Fahrweise mit Haupt- und Nachreaktor wird im Nachreaktor der Katalysator weit weniger stark beansprucht und demzufolge weniger stark deaktiviert. Eine dennoch von Zeit zu Zeit notwendige Reaktivierung, wie z. B. in der WO 02/22259 beschrieben, ist jedoch hier nicht so einfach wie beim Hauptreaktor möglich. Wegen der bevorzugten Ausführung als adiabatischer Reaktor kann die bei der Regenerierung freigesetzte Wärme nicht abgeführt werden, was die Schädigung des Katalysators durch auftretende Temperaturspitzen zur Folge hätte.

Es war daher die Aufgabe gestellt, ein Verfahren zur Verfügung zu stellen, mit dem die katalytische Aktivität des Nachreaktors über sehr lange Betriebszeiten aufrechterhalten werden kann, ohne Abstriche bei der Propylenoxid-Selektivität hinnehmen zu müssen.

Um genau dieses Problem im Hauptreaktor zu lösen, wurde bereits vorgeschlagen, den pH-Wert des Zulaufs und die Reaktortemperatur zu kontrollieren (WO 01/10855). Während eine Änderung des pH-Wertes auch für den Nachreaktor vorteilhaft sein kann, gestaltet sich die Kontrolle der Reaktortemperatur als schwierig, da bei einem adiabatisch geführten Reaktor lediglich die Eingangstemperatur kontrolliert werden kann. Die mindestens zweifache Ausführung des Nachreaktors ist weniger wirtschaftlich, da ein zweiter Reaktor nur selten in Betrieb genommen würde.

In der EP 1 266 892 A1 wird ein Verfahren zur Herstellung von Oxiranen mit Hilfe von Hydroperoxiden in Anwesenheit eines Festbettkatalysators beschrieben. Dabei wird offenbart, dass die Reaktion eine exotherme Reaktion ist und deswegen die plötzliche Entwicklung von Hitze im Festbettreaktor die Aktivität des Katalysators herabsetzen kann. Das Problem des Aktivitätsverlusts wird gemäß EP 1 266 892 A1 dadurch behoben, dass das Reaktionsgemisch, bestehend aus Olefin und Hydroperoxid, in einem Festbett-Flowreaktor über eine Vielzahl von Festbettkatalysatoren geführt wird.

Die Aufgabe konnte dadurch gelöst werden, dass man den Nachreaktor mit mindestens zwei Einspeisestellen für das Reaktionsgemisch, das als Edukte wenigstens die organische Verbindung und das Hydroperoxid sowie das Lösungsmittel umfasst, und/oder mindestens zwei Ablaufstellen für das Produkt ausführt, wobei die Einspeise- oder Ablaufstellenstellen entlang des Reaktors verteilt sind und der Reaktor vorzugsweise weitgehend adiabatisch geführt wird.

Gegenstand der Erfindung ist somit ein Verfahren zur Herstellung von Oxiranen durch Umsetzung einer organischen Verbindung mit einem Hydroperoxid in Anwesenheit eines Lösungsmittels und eines Katalysators, umfassend wenigstens die Schritte (i) bis (iii):
(i) Umsetzung des Hydroperoxids mit der organischen Verbindung unter Erhalt eines Produktgemisches, umfassend die umgesetzte organische Verbindung und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit der organischen Verbindung,

dadurch gekennzeichnet, dass die Umsetzung in den Schritten (i) und (iii) in zwei getrennten Reaktoren und in Schritt (iii) in einem adiabatischen Rohrreaktor erfolgt, der mindestens zwei Einspeisestellen für das Reaktionsgemisch, das wenigstens die organische Verbindung, das Hydroperoxid und das Lösungsmittel umfasst, oder mindestens zwei Ablaufstellen für das Produktgemisch, oder mindestens zwei Einspeise- und mindestens zwei Ablaufstellen besitzt, wobei mindestens eine Einspeisestelle am Boden des Reaktors angebracht ist, mindestens eine Ablaufstelle am Kopf des Reaktors angebracht ist und mindestens eine Einspeise- oder Ablaufstelle oder Einspeise- und Ablaufstelle an der Seite des Reaktors angebracht ist/sind.

Mit dieser Ausführungsform lässt sich die katalytische Aktivität im Nachreaktor im Vergleich zu der beim Stand der Technik beschriebenen Ausführung beträchtlich verlängern. Es können Standzeiten erreicht werden, die mindestens dreimal so hoch sind wie bei einem Nachreaktor, wie er beim Stand der Technik verwendet wird. Dieses Ergebnis war nicht vorhersehbar und ist deshalb als überraschend zu bezeichnen. So werden beim erfindungsgemäßen Verfahren zur Herstellung von Oxiranen unter Verwendung eines Nachreaktors mit mehreren Einspeisestellen aufwendige Regenerierungsmaßnahmen des Katalysators auf ein Minimum begrenzt sowie eine doppelte Ausführung des besagten Nachreaktors überflüssig. Es ist ausreichend, Regenerierungsmaßnahmen dann durchzuführen, wenn die gesamte Anlage beispielsweise wegen allgemeiner Wartungsarbeiten abgestellt ist. Für die industrielle Fertigung ist somit das neue erfindungsgemäße Verfahren außerordentlich günstig.

Unter dem Begriff Rohrreaktor der vorliegenden Erfindung wird dabei ein Strömungsrohr verstanden, bei dem der Reaktionsraum von einem zylindrischen Rohr gebildet wird, dessen Länge vorzugsweise sehr viel größer als der Durchmesser ist. Die Reaktionspartner und Lösungsmittel treten zusammen durch mindestens eine der mindestens zwei Einspeisestellen auf der einen Seite des Rohres ein, während auf der anderen Seite ein Gemisch, welches aus dem Oxiran als Reaktionsprodukt, nicht umgesetzten Edukten, dem Lösungsmittel und weiteren Komponenten, z. B. bei der Reaktion gebildeten Nebenprodukten, besteht, das Rohr über eine oder mehrere Ablaufstellen verlässt.

Der Rohrreaktor kann mit vertikaler Anordnung wie auch mit horizontaler Anordnung der Längsachse verwendet werden. Vorzugsweise ist seine Achse jedoch vertikal angeordnet.

Von den mindestens zwei Einspeisestellen ist dabei mindestens eine am Boden des Reaktors angebracht.

Entlang des Reaktors können beliebig viele weitere Einspeisestellen verwendet werden. Es ist jedoch im Allgemeinen ausreichend, maximal 10 Einspeisestellen zu verwenden. Vorzugsweise werden maximal 5 Einspeisestellen verwendet.

Wird der Reaktor mit mindestens 2 Ablaufstellen ausgeführt, so befindet sich dabei mindestens eine der Ablaufstellen an der Seite des Reaktors und mindestens eine Ablaufstelle am Kopf des Reaktors. Weitere Ablaufstellen können entlang des Reaktors verteilt werden. Es können beliebig viele Ablaufstellen verwendet werden. Es ist jedoch im Allgemeinen ausreichend, maximal 10 Ablaufstellen zu verwenden. Vorzugsweise werden maximal 5 Ablaufstellen verwendet.

Mit diesen Anordnungen können bereits bezüglich der katalytischen Aktivität ausgezeichnete Standzeiten des Nachreaktors erreicht werden.

Demzufolge ist in einer bevorzugten Ausführung das erfindungsgemäße Verfahren dadurch gekennzeichnet, dass der Rohrreaktor mindestens eines der Merkmale (a), (e) und (f) aufweist:
(a) seine Längsachse ist vertikal angeordnet,
(e) die Zahl der Einspeisestellen beträgt maximal 10,
(f) die Zahl der Ablaufstellen beträgt maximal 10.

Gemäß dem erfindungsgemäßen Verfahren wird der Rohrreaktor als Festbettreaktor betrieben, d. h. er enthält die Katalysatormasse, an der die Umsetzung der Reaktanden stattfindet. Der Festbettreaktor kann dabei als Vollraumreaktor ausgeführt sein. Er besteht dann im Allgemeinen aus einem einzigen senkrecht stehenden Rohr, in welchem die Katalysatormasse ohne Unterbrechung angeordnet ist.

Es ist jedoch auch möglich, ihn in Form eines Röhrenreaktors auszuführen. Bei dieser Anordnung wird der Vollraumreaktor in eine Anzahl dünnerer Rohre aufgeteilt.

Der Rohrreaktor kann jedoch auch als Abschnittsreaktor ausgeführt werden. In dieser Ausführung ist die gesamte Katalysatormasse auf zwei oder mehr Schichten aufgeteilt.

Die Reaktionsführung im Nachreaktor erfolgt wegen der exothermen Reaktion zwischen dem in der Stufe (ii) abgetrennten Hydroperoxid und der organischen Verbindung unter adiabatischer Reaktionsführung, die einen gut zu kontrollierenden Verlauf gewährleistet.

Der Nachreaktor kann nach dem erfindungsgemäßen Verfahren mit dem Reaktionsgemisch, nachfolgend auch als Feed bezeichnet, umfassend wenigstens die organische Verbindung und das Hydroperoxid als Reaktanden sowie das Lösungsmittel, nach unterschiedlichen Ausführungsformen beschickt werden.

In einer bevorzugten Ausführungsform wird der gesamte Feed auf alle Einspeisestellen gleichzeitig verteilt und über diese in den Rohrreaktor eingeführt.

Demzufolge ist in dieser Ausführungsform das erfindungsgemäße Verfahren dadurch gekennzeichnet, dass das Reaktionsgemisch dem Nachreaktor über alle Einspeisestellen gleichzeitig zugeführt wird.

In einer weiteren bevorzugten Ausführungsform wird jedoch der Feed zunächst ausschließlich durch eine Einspeisestelle dem Nachreaktor zugeführt. Diese Variante findet vorzugsweise beim Anfahren des Nachreaktors Verwendung. Dabei wird der Feed dem Reaktor vorzugsweise durch die oberste Einspeisestelle solange zugeführt, bis der Umsatz nicht mehr ausreichend hoch ist. Dies kann beispielsweise bereits dann der Fall sein, wenn der Hydroperoxid-Umsatz unter 99,5 % gesunken ist. Danach wird der Feed auf die nächst niedriger angebrachte Einspeisestelle geführt. Wird auch hier der Umsatz zu gering, kann die nächste Einspeisestelle verwendet werden. Nach Bedarf kann dann zur nächsten Einspeisestelle übergegangen werden, usw..

Diese bevorzugte Ausführungsform des erfindungsgemäße Verfahren ist insbesondere dadurch gekennzeichnet, dass das Reaktionsgemisch dem Nachreaktor über die oberste Einspeisestelle zugeführt wird, wobei nach Absinken des Hydroperoxid-Umsatzes auf einen vorher definierten Schwellenwert die nächst niedriger angebrachte Einspeisestelle verwendet wird.

Eine weitere Ausführungsform des erfindungsgemäße Verfahren ist auch dadurch gekennzeichnet, dass das Produktgemisch über die unterste Ablaufslelle abgezogen wird, wobei nach Absinken des Hydroperoxid-Umsatzes auf einen vorher definierten Schwellenwert die nächst höhere Ablaufstelle verwendet wird.

Beide vorstehend beschriebene Ausführungsformen können auch kombiniert werden. Diese bevorzugte Ausführungsform des erfindungsgemäße Verfahren ist dann dadurch gekennzeichnet, dass das Reaktionsgemisch dem Nachreaktor über die oberste Einspeisestelle zugeführt und das Produktgemisch über die unterste Abzugsstelle abgezogen wird, wobei nach Absinken des Hydroperoxid-Umsatzes auf einen vorher definierten Schwellenwert die nächst niedriger angebrachte Einspeisestelle und die nächst höhere Ablaufstelle verwendet werden.

In einer weiteren speziellen Ausführungsform wird der senkrecht aufgestellte Reaktor von unten nach oben mit Flüssigkeit durchströmt, um die Bildung von Gaspolstern zu vermeiden. Gaspolster können beispielsweise bei der Zersetzung von Hydroperoxiden entstehen. Ein bekanntes Beispiel ist die Zersetzung von Wasserstoffperoxid zu Wasser und Sauerstoff. Solche Gaspolster müssen vermieden werden, da sie ein Sicherheitsrisiko darstellen. Auch können solche Gaspolster verhindern, dass sich der Feed gleichmäßig im Festbett verteilen kann, was für eine konstante Reaktionsführung außerordentlich nachteilig ist.

In dieser Ausführungsform wird zur Vermeidung besagter Gaspolster durch die unterste Einspeisestelle, die sich vorzugsweise am Boden des Reaktors befindet, als Flüssigkeit eine Teilmenge des Feeds eingeleitet. Beispielsweise können ca. 5 Gew.-% des Feeds verwendet werden. Es ist jedoch auch möglich, als Flüssigkeit das eingesetzte Lösungsmittel zu verwenden. Bevorzugt wird dieses in einer Menge von bis zu ca. 5 Gew.-% bezogen auf die Feedmenge verwendet.

Nach dieser weiteren bevorzugten Ausführungsform ist das erfindungsgemäße Verfahren dadurch gekennzeichnet, dass die Hauptmenge des Reaktionsgemischs über die oberste Einspeisestelle zugeführt wird, wobei nach Absinken des Hydroperoxid-Umsatzes auf einen vorher definierten Schwellenwert die nächst niedriger angebrachte Einspeisestelle verwendet wird, und gleichzeitig eine Teilmenge des Reaktionsgemisches oder des Lösungsmittels an der untersten Einspeisestelle des Reaktors eingespeist wird.

Vorzugsweise verfügt jede der Einspeisestellen über eine geeignete Vorrichtung, mit deren Hilfe der Feed gleichmäßig über den Reaktorquerschnitt verteilt werden kann.

Im Allgemeinen sind die im erfindungsgemäßen Verfahren beschriebenen Maßnahmen für die vorteilhafte Erhöhung der Standzeit des Katalysators im Nachreaktor ausreichend. Selbstverständlich können jedoch besagte Maßnahmen durch eine variable Einspeisetemperatur des Feeds in den Reaktor oder mit einer Steuerung des pH-Werts unterstützt werden, sofern dies mit einem einfachen und wirtschaftlich vertretbaren Aufwand verbunden ist. Dazu können die Maßnahmen, die in der WO 01/10855 vordringlich für den Hauptreaktor beschrieben sind, auch in analoger Weise auf den Nachreaktor übertragen werden.

So ist es beispielsweise denkbar, zur Steuerung des pH-Wertes dem Feed mindestens eine saure oder mindestens eine basische Verbindung oder ein Gemisch aus zwei oder mehr davon zuzugeben, wobei diese vor der Zugabe gegebenenfalls in einem geeigneten Lösungsmittel oder Lösungsmittelgemisch gelöst werden können.

Der pH-Wert des Feeds kann auch über die Eduktströme, aus denen sich der Feed zusammensetzt und die im Verfahren kontinuierlich in den Feed einfließen, verändert werden. Beispielsweise kann der pH-Wert eines Lösungsmittelstroms oder eines Eduktstroms, der die organische Verbindung enthält, oder auch eines Eduktstroms, der sowohl Lösungsmittel als auch organische Verbindung enthält, vor Zugabe zum Feed verändert und über diesen Weg der pH-Wert des Reaktionsgemisches im Nachreaktor beeinflusst werden.

Der pH-Wert des Reaktionsmediums im Nachreaktor kann während der Umsetzung auch über den pH-Wert der Hydroperoxidlösung, die in den Feed einfließt, beeinflusst werden.

Wie bereits vorstehend erläutert, ist die Anpassung der Temperatur weniger gut zur Unterstützung des erfindungsgemäßen Verfahrens geeignet. Jedoch kann die Temperatur des Reaktionsgemisches im Nachreaktor auch über die Temperatur des Feed-Stroms beeinflusst werden.

Zur Unterstützung des erfindungsgemäßen Verfahrens ist es prinzipiell möglich, während der Umsetzung des Hydroperoxids mit der organischen Verbindung im Nachreaktor die Temperatur und den pH-Wert des Feeds getrennt voneinander zu variieren. Weiter ist es aber auch möglich, pH-Wert und Temperatur gleichzeitig zu ändern.

Sollen pH-Wert- und Temperaturänderung zur Unterstützung des erfindungsgemäßen Verfahrens herangezogen werden, wird eine konstante Aktivität des heterogenen Katalysators im Allgemeinen auch dadurch erreicht, dass die Temperatur des Reaktionsmediums im Nachreaktor mit fortschreitender Versuchsdauer erhöht wird. Ob hingegen der pH-Wertes des Reaktionsmediums erhöht oder erniedrigt werden muss, hängt wesentlich von der Art des eingesetzten Katalysator ab. Wird wie in einer bevorzugten Ausführungsform des erfindungsgemäßen Verfahren ein titanhaltiger Silicalit als heterogener Katalysator eingesetzt, beispielsweise der Silicalit TS-1, so muss zur Konstanthaltung der Aktivität des Katalysators im Allgemeinen der pH-Wert im Laufe der Umsetzung erniedrigt werden.

Dabei ist es selbstverständlich denkbar, dass beispielsweise im Laufe der Umsetzung auch eine oder mehrere Temperaturerhöhungen oder eine oder mehrere pH-Wert-Erniedrigungen vorgenommen werden, um die Aktivität und Selektivität des Katalysators an einen vorgegebenen Sollwert anzupassen.

Im Allgemeinen liegt die Temperatur des Feeds bei der Einspeisung im Bereich von 0 bis 120 °C, bevorzugt im Bereich von 10 bis 100 °C und weiter bevorzugt im Bereich von 20 bis 90 °C. Der pH-Wert liegt im Allgemeinen im Bereich von 2 bis 6 und besonders bevorzugt im Bereich von 3 bis 6. Im Dauerbetrieb können zur Unterstützung des erfindungsgemäßen Verfahrens die Temperatur auch um vorzugsweise 2 °C/Tag oder weniger, weiter bevorzugt um 0,2 bis 1,0 °C/Tag und der pH-Wert um vorzugsweise 0,5 Einheiten/Tag oder weniger, weiter bevorzugt um 0,01 bis 0,2 Einheiten/Tag geändert werden. Die im Nachreaktor auftretenden Drücke reichen dabei von 1 bis 100 bar, vorzugsweise von 1 bis 40 bar, besonders bevorzugt von 1 bis 30 bar.

Neben den Parametern Temperatur und pH-Wert des Reaktionsmediums kann im Rahmen der vorliegenden Erfindung zusätzlich auch der Druck, unter dem die Umsetzung stattfindet, variiert werden. Bevorzugt wird allerdings bei Drücken gearbeitet, unter denen keine Gasphase vorliegt.

Demzufolge ist das erfindungsgemäße Verfahren auch dadurch gekennzeichnet, dass bei der Einspeisung in den Rohrreaktor der pH-Wert des Reaktionsgemisches 2 bis 6 und die Temperatur 0 bis 120 °C sowie der Druck im Rohrreaktor 1 bis 100 bar beträgt.

Die Konzentration der umzusetzenden organischen Verbindung wird im Allgemeinen so gewählt, dass im Feed das molare Verhältnis von umzusctzender organischer Verbindung zu Hydroperoxid bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt.

Die Umsetzung wird in diesem Fall bei einem Druck, einer Temperatur und einer Verweilzeit des Reaktionsgutes so durchgeführt, dass Hydroperoxidumsätze erzielt werden, die im Allgemeinen über 90%, bevorzugt über 92% und besonders bevorzugt im Bereich von 95 bis 99,5% liegen.

Die Verweilzeiten des Reaktionsgemisches im Nachreaktor richten sich dabei im Wesentlichen nach den gewünschten Umsätzen. Im Allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Für das erfindungsgemäße Verfahren zur Herstellung von Oxiranen können die aus dem Stand der Technik bekannten Edukte verwendet werden.

Bevorzugt werden organische Verbindungen umgesetzt, die mindestens eine C-C-Doppelbindung aufweisen. Als Beispiele für solche organischen Verbindungen mit mindestens einer C-C-Doppelbindung seien folgende Alkene genannt:

Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicoscne, Tri- und Tetrapropen, Polybutadiene, Polyisobutene, Isoprene, Terpene, Geraniol, Linalool, Linalylacetat, Methylencyclopropan, Cyclopenten, Cyclohexen, Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren, wie z.B. Acrylsäure, Methacrylsäure, Crotonsäure, Maleinsäure, Vinylessigsäure, sowie ungesättigte Fettsäuren, wie z.B. Ölsäure, Linolsäure, Palmitinsäure, ferner natürlich vorkommende Fette und Öle.

Bevorzugt werden Alkene verwendet, die 2 bis 8 Kohlenstoffatome enthalten. Besonders bevorzugt werden Ethen, Propen und Buten umgesetzt. Insbesondere bevorzugt wird Propen umgesetzt.

Als Hydroperoxid können die bekannten Hydroperoxide, die für die Umsetzung der organischen Verbindung geeignet sind, eingesetzt werden. Beispiele für solche Hydroperoxide sind etwa tert.-Butylhydroperoxid oder Ethylbenzolhydroperoxid. Bevorzugt wird als Hydroperoxid für die Oxiransynthese Wasserstoffperoxid eingesetzt, wobei auch eine wässerige Wasserstoffperoxidlösung verwendet werden kann.

Zur Herstellung von Wasserstoffperoxid kann dabei beispielsweise auf das Anthrachinonverfahren zurückgegriffen werden. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmann's Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Die Oxiransynthese durch Umsetzung des Hydroperoxids mit der organischen Verbindung wird in Gegenwart eines Lösungsmittels und eines oder mehrerer geeigneter Katalysatoren durchgeführt. Dabei wird wiederum heterogenen Katalysatoren der Vorzug gegeben.

Es sind alle heterogenen Katalysatoren denkbar, die für die jeweilige Umsetzung geeignet sind. Bevorzugt werden dabei Katalysatoren verwendet, die ein poröses oxidisches Material, wie z. B. einen Zeolithen, umfassen. Vorzugsweise werden Katalysatoren eingesetzt, die als poröses oxidisches Material einen Titan-, Germanium-, Tellur-, Vanadium-, Chrom-, Niob- oder Zirkonium-haltigen Zeolithen umfassen.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind Ti-Zeolithe mit MFI-, MEL- oder MFI/MEL-Mischstruktur anzusehen. Ganz besonders bevorzugt sind im Einzelnen die Titan-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur.

Sehr günstig ist die Verwendung eines heterogenen Katalysator, der das Titan-haltige Silikalit TS-1 umfasst.

Dabei ist es auch möglich, als Katalysator das poröse oxidische Material an sich zu verwenden. Selbstverständlich ist es jedoch auch möglich, als Katalysator einen Formkörper einzusetzen, der das poröse oxidische Material umfasst. Dabei können zur Herstellung des Formkörpers, ausgehend von dem porösen oxidischen Material, alle Verfahren gemäß dem Stand der Technik eingesetzt werden.

Vor, während oder nach dem einen oder mehreren Formgebungsschritten in diesen Verfahren können auf das Katalysatormaterial Edelmetalle in Form geeigneter Edelmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben.

Die Formkörper können auch konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise vorstehend beschrieben.

Bei Verwendung eines oder auch mehrer Formkörper als Katalysator kann dieser nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Auf Katalysatoren vom Zeolith-Typ kann diese Methode besonders gut angewendet werden. Besagtes Regenerierungsverfahren ist in der DE-A 197 23 949.8 beschrieben. Femer können auch die dort beim Standes der Technik angegebenen Regenerierungsverfahren eingesetzt werden.

Als Lösungsmittel können vorzugsweise alle Lösungsmittel verwendet werden, die die in die Oxiransynthese eingesetzten Edukte ganz oder wenigstens teilweise lösen. Beispielsweise können verwendet werden Wasser; Alkohole, bevorzugt niedere Alkohole, weiter bevorzugt Alkohole mit weniger als 6 Kohlenstoffatomen wie beispielsweise Methanol, Ethanol, Propanole, Butanole, Pentanole, Diole oder Polyole, bevorzugt solche mit weniger als 6 Kohlenstoffatomen; Ether, wie beispielsweise Diethylether, Tetrahydrofuran, Dioxan, 1,2-Diethoxyethan, 2-Methoxyethanol; Ester, wie beispielsweise Methylacetat oder Butyrolacton; Amide, wie beispielsweise Dimethylformamid, Dimethylacetamid, N-Methylpyrrolidon; Ketone, wie beispielsweise Aceton; Nitrile, wie beispielsweise Acetonitril; Sulfoxide, wie beispielsweise Dimethylsulfoxid; aliphatische, cycloaliphatische und aromatische Kohlenwasserstoffe, oder Gemische aus zwei oder mehr der vorgenannten Verbindungen.

Bevorzugt werden Alkohole eingesetzt. Dabei ist der Einsatz von Methanol als Lösungsmittel besonders bevorzugt.

Nach dem erfindungsgemäßen Verfahren ist es auch möglich, mehrere organische Verbindungen mit dem Hydroperoxid umzusetzen. Ebenso ist es denkbar, zur Umsetzung mehrere Hydroperoxide zu verwenden. Werden mehrere organische Verbindungen und/oder mehrere Hydroperoxide miteinander in den jeweiligen Stufen umgesetzt, so können in den Mischungen verschiedenartige Produkte, die aus den Umsetzungen resultieren, vorliegen. Falls gewünscht können die entstandenen Oxirane destillativ getrennt werden.

In einer Ausführungsform des erfindungsgemäßen Verfahrens können weiterhin der pH-Wert oder/und die Temperatur des Reaktionsgemisches im Nachreaktor geändert werden.

Soll hierbei der pH-Wert des Reaktionsgemisches durch Änderung des pH-Wertes der Hydroperoxidlösung, die dem Feed zugegeben wird, erreicht werden, so kann beispielsweise derjenige Strom, der das in Stufe (ii) abgetrennt Hydroperoxid umfasst, zur Änderung des pH-Wertes in geeigneter Weise behandelt werden.

Der pH-Wert eines Hydroperoxidstromes, insbesondere der Wasserstoffperoxidlösung, kann dabei nach gängigen Verfahren eingestellt werden. Dabei ist lediglich darauf zu achten, dass bei Zugabe von sauren oder basischen Verbindungen oder bei Zugabe einer Lösung, die saure oder basische Verbindungen umfasst, zur Hydroperoxidlösung die nachfolgende Umsetzung der organischen Verbindung mit Hydroperoxid nicht nachteilig beeinflusst wird. Insbesondere kann der pH-Wert der Hydroperoxidlösung durch Behandlung der Hydroperoxidlösung mit mindestens einem Ionentauscher, durch Zugabe einer sauren, einer basischen oder einer neutralen Verbindung oder eines Gemisches aus zwei oder mehr davon zur Hydroperoxidlösung oder durch eine Kombination dieser Methoden geändert werden.

Hierbei sind prinzipiell sowohl stark basische als auch schwach basische Verbindungen oder sowohl stark saure als auch schwach saure Verbindungen geeignet. Insbesondere kommen folgende Salze in Betracht:

Ammoniumsalze, Alkalisalze, wobei vor allem Lithium-, Natrium- und Kaliumsalze zu nennen sind, sowie Erdalkalisalze. Die Anionen dieser Salze umfassen beispielsweise Halogenide wie beispielsweise Chlorid und Bromid, Nitrat, Sulfat oder Hydroxid sowie die Anionen von Phosphor, Arsen, Antimon und Zinn enthaltenden Säuren, wie z. B. Perchlorat, Phosphat, Hydrogenphosphat, Dihydrogenphosphat, Arsenat und Stannat. Auch andere Anionen, wie beispielsweise Formiat, Acetat, Hydrogencarbonat oder Carbonat, sind denkbar.

Als Beispiele seien unter anderem Lithiumchlorid, Lithiumbromid, Natriumbromid, Lithiumnitrat, Natriumnitrat, Kaliumnitrat, Lithiumsulfat, Natriumsulfat, Kaliumsulfat, Natriumhydroxid, Kaliumhydroxid, Ammoniumhydroxid, Natriumcarbonat, Natriumhydrogencarbonat, Kaliumcarbonat, Lithiumcarbonat, Kaliumhydrogencarbonat, Lithiumhydrogencarbonat und Kaliumhydrogenphosphat sowie Lithium-, Magnesium-, Calcium-, Barium oder Ammoniumacetat genannt. Ebenfalls zu nennen sind Carboxylate von Carbonsäuren, insbesondere von Carbonsäuren mit 1 bis 10 Kohlenstoffatomen, sowie Alkoholate von Alkoholen mit 1 bis 10 Kohlenstoffatomen. Weitere Beispiele sind unter anderem Ammoniumdihydrogenphosphat, Natriumdihydrogenphosphat, Kaliumdihydrogenphosphat, Dinatriumdihydrogenpyrophosphat.

Als Ionentauscher lassen sich hierbei grundsätzlich alle dem Fachmann bekannten Ionenaustauscher einsetzen, beispielsweise organische Ionenaustauscher, etwa auf Polystyrolbasis, oder anorganische Ionenaustauscher, etwa Hydrotalcite sowie andere Schichtsilikate, die austauschbare Carbonat-, Hydrogencarbonat- oder Hydroxidgruppen enthalten können.

Beispiele für die im Rahmen der vorliegenden Erfindung besonders bevorzugten basischen Ionenaustauscher sind Polystyrolharze mit tertiären Amingruppen, etwa die kommerziell erhältlichen Anionenaustauscher Lewatit® MP62 und Lewatit® MP 63 sowie Dowex® MWA/1 und Dowex® AMW-500. Darüber hinaus ist auch die Verwendung von etwa quartäre Ammoniumgruppen enthaltenden Polystyrolharzen mit Hydroxid-Gegenionen denkbar. Beispielhaft seien hierbei die kommerziell erhältlichen Austauscher Lewatit® OC-1950 sowie Dowex® 1, Dowex® 2, Dowex® 11, Dowex® 21K und Dowex® 550A genannt.

Es ist jedoch auch möglich, die aufgeführten Verbindungen dem Feed vor Einspeisung in den Nachreaktor direkt oder in einem Lösungsmittel gelöst zuzusetzen.

Die Temperatur des Feeds kann beispielsweise über die Temperatur mindestens eines Eduktstromes gesteuert werden, der dem Feed zugeführt wird.

Als Hauptreaktor können für die Oxiran-Synthese des erfindungsgemäßen Verfahrens selbstverständlich alle denkbaren, für die jeweiligen Reaktionen am besten geeigneten Reaktoren eingesetzt werden. Dabei ist für die Oxiran-Synthese ein Reaktor nicht auf einen einzelnen Behälter beschränkt. Vielmehr ist es auch möglich, beispielsweise eine Rührkesselkaskade einzusetzen. Vorzugsweise werden, wie in der WO 01/72729 beschrieben, mindestens zwei parallel geschaltete Reaktoren eingesetzt.

Bevorzugt wird für die Oxiran-Synthese als Hauptreaktor ein Festbettreaktor verwendet. Weiter bevorzugt wird als Festbettreaktor ein Festbettrohrreaktor eingesetzt.

Insbesondere wird für das erfindungsgemäße Verfahren zur Herstellung von Oxiranen als Reaktor für die Stufe (i) ein isothermer Festbettreaktor und für die Stufe (iii) ein adiabatischer Festbettrohrreaktor verwendet.

Die Abtrennung des Hydroperoxids in Stufe (ii) erfolgt vorzugsweise destillativ in einer Kolonne.

Bevorzugt wird das Oxiran in derselben Abtrennvorrichtung neben dem Hydroperoxid direkt aus der Reaktionsmischung abgetrennt. Bei dieser destillativen Zwischenabtrennung wird das Oxiran dann über den Kolonnenkopf der Mischung entnommen. Über den Seitenabzug der Kolonne wird das Hydroperoxid abgetrennt. In einer weiteren Ausführung des Verfahren ist es jedoch auch möglich, das Hydroperoxid nicht über den Seitenabzug der Kolonne, sondern über den Sumpf im Kolonnenboden aus der Mischung abzutrennen. Es enthält gewöhnlich noch Wasser und das Lösungsmittel, das für die Umsetzung verwendet wird.

Das über den Kolonnenkopf abdestillierte Oxiran ist mit unter den Destillationsbedingungen flüchtigen Niedrigsiedern verunreinigt, beispielsweise mit nicht umgesetzter organischer Verbindung, Wasser oder Lösungsmittel. Es muss dann gewöhnlich einem Reinigungsschritt unterzogen werden, etwa einer weiteren Destillation in einer Kolonne, die mit der als Abtrenneinrichtung verwendeten Kolonne in Reihe geschaltet ist, um es in der für die weitere Anwendung notwendigen Reinheit zu erhalten.

Auch das in Stufe (iii) aus dem Nachreaktor erhaltene Produktgemisch muss zur Gewinnung des Oxirans gewöhnlich einem Destillalionsschritt unterworfen werden. Dazu kann es analog zu vorstehend beschriebener Vorgehensweise in eine Destillationsapparatur überführt werden. Es ist jedoch auch möglich, es mit dem aus Stufe (ii) erhaltenen Produktgemisch zu vereinen, und daraus durch Destillation das Oxiran zu erhalten.

In einer besonders bevorzugten Ausführung des erfindungsgemäßen Verfahrens wird als organische Verbindung Propylen und als Hydroperoxid Wasserstoffperoxid eingesetzt, sowie die Umsetzung in Methanol als Lösungsmittel an einem heterogenen Katalysator umfassend einen Titan-haltigen Zeolith durchgeführt. Somit betrifft in einer besonders bevorzugten Ausführung das erfindungsgemäße Verfahren die Herstellung von Propylenoxid unter Verwendung eines Nachreaktors mit mehreren Einspeisestellen.

Hierbei erreicht der Wasserstoffperoxid-Umsatz in Stufe (i) ca. 85 % bis 90 % und in Stufe (iii) ca. 95 % bezogen auf Stufe (ii). In der Summe kann über beide Stufen ein Wasserstoffperoxidumsatz von ca. 99 % bei einer Propylenoxid-Selektivität von ca. 94 - 95 % erreicht werden.

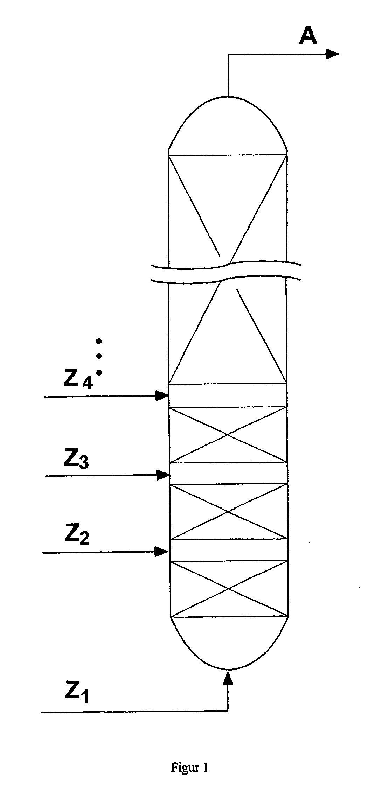

Die Erfindung soll nun beispielhaft anhand der Figuren 1 bis 3 erläutert werden.

Dabei zeigt Figur 1 einen Reaktor, der eine Einspeisestelle Z₁ am Boden, eine Ablaufstelle A am Kopf und weitere Einspeisestellen Z₂, Z₃, Z₄, ... an der Seite besitzt.

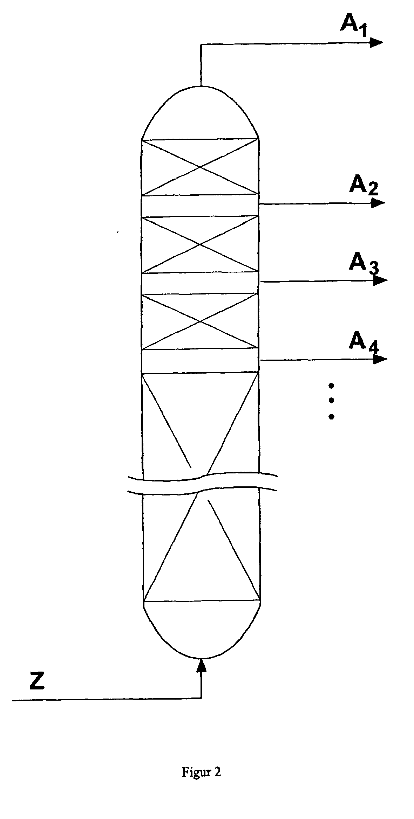

Möglich ist aber auch eine Anordnung wie in Figur 2 skizziert. Dabei befindet sich die Einspeisestelle Z am Boden des Reaktors, eine Ablaufstelle A₁ am Kopf, und weitere Ablaufstellen A₂, A₃, A₄, ... an der Seite des Reaktors.

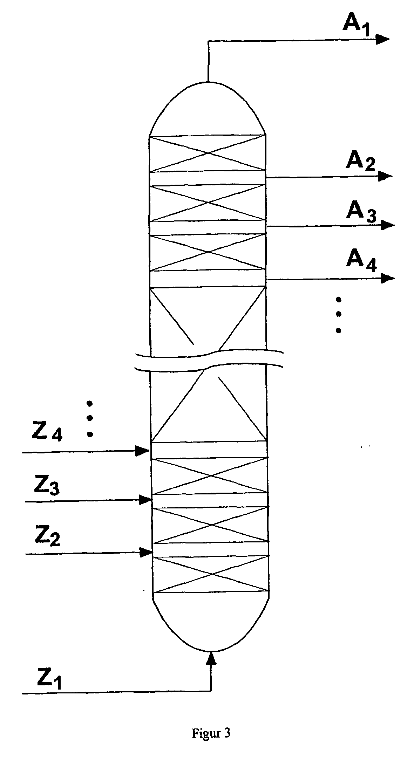

Figur 3 zeigt einen Reaktor, der eine Einspeisestelle Z₁ am Boden, eine Ablaufstelle A₁ am Kopf und weitere Einspeisestellen Z₂, Z₃, Z₄, ... und Ablaufstellen A₂, A₃, A₄, ... an der Seite besitzt.

Der Feed, enthaltend die Reaktanden, wird über eine oder mehrere der Einspeisestellen Z, Z₁, Z₂, Z₃, Z₄, ... in den Reaktor gebracht, und das Produktgemisch verlässt ihn über eine oder mehrere der Ablaufstellen A, A₁, A₂, A₃, A₄, .... Durch geeignete Wahl von Einlass- und Ablaufstellen können wie vorstehend beschrieben im jeweiligen Reaktor Reaktionszonen so ausgewählt werden, dass bezüglich der katalytischen Aktivität stets weitgehend konstante Bedingungen erhalten werden.

Waagrechte und diagonale oder diagonal angedeutete Linien in den Reaktoren symbolisieren Festbettschüttungen oder Packungen oder Schichten mit Katalysatormasse.

## Patentansprüche

1. Verfahren zur Herstellung von Oxiranen durch Umsetzung einer organischen Verbindung mit einem Hydroperoxid in Anwesenheit eines Lösungsmittels und eines Katalysators, umfassend wenigstens die Schritte (i) bis (iii):
(i) Umsetzung des Hydroperoxids mit der organischen Verbindung unter Erhalt eines Produktgemisches, umfassend die umgesetzte organische Verbindung und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit der organischen Verbindung,
wobei die Umsetzung in den Schritten (i) und (iii) in zwei getrennten Reaktoren und in Schritt (iii) in einem adiabatischen Rohrreaktor erfolgt, der mindestens zwei Einspeisestellen für das Reaktionsgemisch, das wenigstens die organische Verbindung, das Hydroperoxid und das Lösungsmittel umfasst, oder mindestens zwei Ablaufstellen für das Produktgemisch, oder mindestens zwei Einspeise- und mindestens zwei Ablaufstellen besitzt, wobei mindestens eine Einspeisestelle am Boden des Reaktors angebracht ist, mindestens eine Ablaufstelle am Kopf des Reaktors angebracht ist und mindestens eine Einspeise- oder Ablaufstelle oder Einspeise- und Ablaufstelle an der Seite des Reaktors angebracht ist/sind.

2. Verfahren nach Anspruch 1, **dadurch gekennzeichnet, dass** in Stufe (i) ein isothermer Festbettreaktor und in Stufe (iii) als Rohrreaktor ein resibettreaktor verwendet wird.

3. Verfahren nach Anspruch 1 oder 2, **dadurch gekennzeichnet, dass** der Rohrreaktor mindestens eines der Merkmale (a), (e) und (f) aufweist:
(a) seine Längsachse ist vertikal angeordnet,
(e) die Zahl der Einspeisestellen beträgt maximal 10,
(f) die Zahl der Ablaufstellen beträgt maximal 10.

4. Verfahren nach einem der Ansprüche 1 bis 3, **dadurch gekennzeichnet, dass** das Reaktionsgemisch dem Rohrreaktor über alle Einspeisestellen gleichzeitig zugeführt wird.

5. Verfahren nach einem der Ansprüche 1 bis 3, **dadurch gekennzeichnet, dass** das Reaktionsgemisch dem Rohrreaktor ausschließlich über die oberste Einspeisestelle zugeführt wird, wobei nach Absinken des Hydroperoxid-Umsatzes bis auf einen vorher definierten Schwellenwert das Reaktionsgemisch über die nächst niedriger angebrachte Einspeisestelle zugeführt wird.

6. Verfahren nach einem der Ansprüche 1 bis 5, **dadurch gekennzeichnet, dass** das Produkgemisch dem Rohrreaktor ausschließlich über die unterste Ablaufstelle entnommen wird, wobei nach Absinken des Hydroperoxid-Umsatzes bis auf einen vorher definierten Schwellenwert das Produktgemisch über die nächst höher angebrachte Ablaufstelle entnommen wird.

7. Verfahren nach einem der Ansprüche 1 bis 6, **dadurch gekennzeichnet, dass** gleichzeitig mit dem Reaktionsgemisch eine Teilmenge des Reaktionsgemisches oder des Lösungsmittels an der untersten Einspeisestelle des Rohrreaktors eingespeist wird.

8. Verfahren nach einem der Ansprüche 1 bis 7, **dadurch gekennzeichnet, dass** jede Einspeisestelle über eine Vorrichtung verfügt, über die das Reaktionsgemisch über den gesamten Querschnitt des Rohrreaktors gleichmäßig verteilt wird.

9. Verfahren nach einem der Ansprüche 1 bis 8, **dadurch gekennzeichnet, dass** bei der Einspeisung in den Rohrreaktor der pH-Wert des Reaktionsgemisches 2 bis 6 und die Temperatur 0 bis 120 °C sowie der Druck im Rohrreaktor 1 bis 100 bar beträgt.

10. Verfahren nach einem der Ansprüche 1 bis 9, **dadurch gekennzeichnet, dass** als organische Verbindung Propylen und als Hydroperoxid Wasserstoffperoxid eingesetzt wird, das Oxiran Propylenoxid ist, und die Umsetzung in Methanol als Lösungsmittel an einem heterogenen Katalysator, der ein Zeolith umfasst, durchgeführt wird.

11. Verfahren nach Anspruch 10, **dadurch gekennzeichnet, dass** der Zeolith TS-1 ist.

## Claims

1. A process for preparing oxiranes by reacting an organic compound with a hydroperoxide in the presence of a solvent and a catalyst, which comprises at least the steps (i) to (iii):
(i) reaction of the hydroperoxide with the organic compound to give a product mixture comprising the reacted organic compound and unreacted hydroperoxide,
(ii) separation of the unreacted hydroperoxide from the mixture resulting from step (i),
(iii) reaction of the hydroperoxide which has been separated off in step (ii) with the organic compound,
wherein the reaction in steps (i) and (iii) is carried out in two separate reactors and in step (iii) is carried out in an adiabatic tube reactor which has at least two feed points for the reaction mixture comprising at least the organic compound, the hydroperoxide and the solvent or at least two outlets for the product mixture, or at least two feed points and at least two outlets, with at least one feed point being located at the bottom of the reactor, at least one outlet being located at the top of the reactor and at least one feed point or outlet or feed point and outlet being located at the side of the reactor.

2. The process according to claim 1, wherein an isothermal fixed-bed reactor is used in step (i) and a fixed-bed reactor is used as tube reactor in step (iii).

3. The process according to claim 1 or 2, wherein the tube reactor has at least one of the features (a), (e) and (f):
(a) its longitudinal axis is arranged vertically,
(e) the number of feed points is not more than 10,
(f) the number of outlets is not more than 10.

4. The process according to any of claims 1 to 3, wherein the reaction mixture is fed into the tube reactor simultaneously via all feed points.

5. The process according to any of claims 1 to 3, wherein the reaction mixture is fed into the tube reactor exclusively via the uppermost feed point and after the hydroperoxide conversion has dropped to a predefined threshold value, the reaction mixture is fed in via the next lower feed point.

6. The process according to any of claims 1 to 5, wherein the product mixture is taken from the tube reactor exclusively via the bottommost outlet and when the hydroperoxide conversion has dropped to a previously defined threshold value, the product mixture is taken off via the next higher outlet.

7. The process according to any of claims 1 to 6, wherein part of the reaction mixture or of the solvent is fed in simultaneously with the reaction mixture at the bottommost feed point of the tube reactor.

8. The process according to any of claims 1 to 7, wherein each feed point is provided with a device by means of which the reaction mixture is uniformly distributed over the entire cross section of the tube reactor.

9. The process according to any of claims 1 to 8, wherein the reaction mixture fed into the tube reactor has a pH of from 2 to 6 and a temperature of from 0 to 120°C and the pressure in the tube reactor is from 1 to 100 bar.

10. The process according to any of claims 1 to 9, wherein propylene is used as organic compound and hydrogen peroxide is used as hydroperoxide, the oxirane is propylene oxide and the reaction is carried out in methanol as solvent over a heterogeneous catalyst comprising a zeolite.

11. The process according to claim 10, wherein the zeolite is TS-1.

## Revendications

1. Procédé de préparation d'oxiranes par transformation d'un composé organique avec un hydroperoxyde en présence d'un solvant et d'un catalyseur, comprenant au moins les étapes (i) à (iii) :
(i) transformation de l'hydroperoxyde avec le composé organique sous obtention d'un mélange de produits, comprenant le composé organique transformé et l'hydroperoxyde non transformé;
(ii) séparation de l'hydroperoxyde non transformé du mélange résultant de l'étape (i) ;
(iii) transformation de l'hydroperoxyde séparé de l'étape (ii) avec le composé organique,
dans lequel la transformation dans les étapes (i) et (iii) s'effectue dans deux réacteurs séparés et dans l'étape (iii) dans un réacteur tubulaire adiabatique qui possède au moins deux points d'alimentation pour le mélange réactionnel, comprenant au moins le composé organique, l'hydroperoxyde et le solvant, ou au moins deux points d'écoulement du mélange de produits, ou au moins deux points d'alimentation et au moins deux points d'écoulement, au moins un point d'alimentation étant réalisé au fond du réacteur, au moins un point d'écoulement étant réalisé à la tête du réacteur et au moins un point d'alimentation ou d'écoulement ou un point d'alimentation et d'écoulement étant situé/s sur le côté du réacteur.

2. Procédé selon la revendication 1, **caractérisé en ce que** dans l'étape (i), on utilise un réacteur à lit fixe isotherme et dans l'étape (iii) on utilise comme réacteur tubulaire un réacteur à lit fixe.

3. Procédé selon l'une des revendications 1 ou 2, **caractérisé en ce que** le réacteur tubulaire présente au moins une des caractéristiques (a), (e) et (f) :
(a) son axe longitudinal est agencé verticalement,
(e) le nombre des points d'alimentation est au maximum de 10,
(f) le nombre des points d'écoulement est au maximum de 10.

4. Procédé selon l'une quelconque des revendications 1 à 3, **caractérisé en ce que** le mélange réactionnel est amené au réacteur tubulaire par tous les points d'alimentation en même temps.

5. Procédé selon l'une quelconque des revendications 1 à 3, **caractérisé en ce que** le mélange réactionnel est amené au réacteur tubulaire exclusivement par le point d'alimentation le plus élevé, le mélange réactionnel étant amené par le point d'alimentation inférieur suivant après la diminution de la conversion de l'hydroperoxyde à une valeur de seuil préalablement définie.

6. Procédé selon l'une quelconque des revendications 1 à 5, **caractérisé en ce que** le mélange de produits est prélevé du réacteur tubulaire exclusivement par le point d'écoulement le plus inférieur, le mélange de produits étant prélevé.par le point d'écoulement supérieur suivant après la diminution de la conversion de l'hydroperoxyde à une valeur de seuil préalablement définie.

7. Procédé selon l'une quelconque des revendications 1 à 6, **caractérisé en ce qu'**en même temps que le mélange réactionnel, une partie du mélange réactionnel ou du solvant est introduit au point d'alimentation le plus bas du réacteur tubulaire.

8. Procédé selon l'une quelconque des revendications 1 à 7, **caractérisé en ce que** chaque point d'alimentation est doté d'un dispositif par le biais duquel le mélange réactionnel est réparti sur toute la section du réacteur tubulaire de manière égale.

9. Procédé selon l'une quelconque des revendications 1 à 8, **caractérisé en ce que** lors de l'alimentation dans le réacteur tubulaire, le pH du mélange réactionnel vaut de 2 à 6 et la température de 0 à 120°C et la pression dans le réacteur tubulaire est de 1 à 100 bars.

10. Procédé selon l'une quelconque des revendications 1 à 9, **caractérisé en ce qu'**on utilise comme composé organique le propylène et comme hydroperoxyde le peroxyde d'hydrogène, l'oxirane est l'oxyde de propylène et la transformation dans le méthanol comme solvant s'effectue sur un catalyseur hétérogène comprenant un zéolithe.

11. Procédé selon la revendication 10, **caractérisé en ce que** le zéolithe est le TS-1.
